# EUROPEAN PATENT APPLICATION

(11) **EP 4 645 337 A1**
(43) Date of publication of application: **05.11.2025**
(21) Application number: 23911590.0
(22) Date of filing: 06.12.2023
(51) Int. Cl.: G16H 50/30

(54) **EVALUATION DEVICE, EVALUATION METHOD, EVALUATION SYSTEM, AND EVALUATION PROGRAM**

(30) Priority: 27.12.2022 JP 2022210756
(71) Applicant: Sunstar Suisse SA, 1163 Etoy (CH)
(72) Inventor: OTA, Anri, Takatsuki-shi, Osaka 569-1195 (JP); SHIMIZU, Sato, Takatsuki-shi, Osaka 569-1195 (JP); INOUE, Masaharu, Takatsuki-shi, Osaka 569-1195 (JP); NAKANISHI, Takashi, Takatsuki-shi, Osaka 569-1195 (JP); KANATA, Takeshi, Takatsuki-shi, Osaka 569-1195 (JP)
(74) Representative: Plasseraud IP
(86) International application number: PCT/JP2023/043619
(87) International publication number: WO 2024/142809

(57) **Abstract**

Provided is an evaluation device and others which are capable of properly evaluating an oral state. An evaluation device (1) includes: an non-practice period identifying section (103) for identifying the length of a non-practice period, which is a period during which a subject of oral state evaluation does not practice predetermined oral care; and an evaluation section (105) for evaluating the oral state of the subject depending on the length of the non-practice period.

## Description

### Technical Field

The present invention relates to an evaluation device and others for evaluating an oral state of a subject.

### Background Art

Techniques for evaluating teeth-brushing practiced by a subject and providing feedback on the quality of the teeth-brushing have conventionally been known. For example, Patent Literature 1 below discloses analyzing brushing data detected by a smart toothbrush which incorporates various sensors and thereby obtaining a brushing evaluation result.

### Citation List

### [Patent Literature]

[Patent Literature 1]
Japanese Translation of PCT International Application, Tokuhyo, No. 2021-519201

### Summary of Invention

### Technical Problem

The technique disclosed in Patent Literature 1 does not consider that risks concerning an oral health state have the characteristic of changing depending on the length of an oral care non-practice period. Thus, said technique is susceptible to improvement in terms of proper evaluation of an oral state.

For example, tartar is formed through three steps which are pellicle formation, plaque formation, and calcification. Tartar, which is difficult to remove via routine teeth-brushing, becomes the matrix of another plaque buildup, thereby creating an environment in which bacteria causing periodontal disease easily grow. Thus, there is a significant difference between the health risks to an oral cavity before and after the start of hardening into tartar. This means that, in regard to a site in which hardening into tartar has started, however high the result of evaluation of a single time of brushing, there is a high possibility that hardening into tartar will proceed and an oral health state will worsen accordingly.

An object of an aspect of the present invention is to provide an evaluation device and others which are capable of properly evaluating an oral state.

### Solution to Problem

In order to solve the above problem, an evaluation device in accordance with an aspect of the present invention includes: a non-practice period identifying section for identifying the length of a non-practice period, which is a period during which a subject of oral state evaluation does not practice predetermined oral care; and an evaluation section for evaluating an oral state of the subject on a basis of the length of the non-practice period.

In order to solve the above problem, an evaluation method in accordance with an aspect of the present invention is an evaluation method carried out by one or more information processing devices, and the evaluation method includes: a non-practice period identifying step of identifying the length of a non-practice period, which is a period during which a subject of oral state evaluation does not practice predetermined oral care; and an evaluation step of calculating an evaluation value indicative of an oral state of the subject depending on the length of the non-practice period.

In order to solve the above problem, an evaluation system in accordance with an aspect of the present invention includes: a record accumulation device for accumulating, with respect to one or more subjects of oral state evaluation, record information indicative of a record of use of a toothbrush by said one or more subjects; an evaluation device for identifying, with respect to said one or more subjects, the length of a non-practice period, which is a period during which said one or more subjects do not practice predetermined oral care, on a basis of the record information, and evaluating an oral state depending on the length of the non-practice period; and a presentation device for presenting feedback information depending on a result of the oral state evaluation.

The evaluation devices in accordance with the aspects of the present invention may be provided by a computer. In that case, an evaluation device control program for causing a computer to operate as the sections (software elements) of the evaluation devices and thereby providing the evaluation devices via the computer and a computer-readable recording medium which has recorded thereon such an evaluation device control program are within the scope of the present invention.

### Advantageous Effects of Invention

With an aspect of the present invention, it is possible to provide an evaluation device and others which are capable of properly evaluating an oral state.

### Brief Description of Drawings

Fig. 1 is a block diagram illustrating an example configuration of the main part of an evaluation device in accordance with an embodiment of the present invention.
Fig. 2 is a representation of an example configuration of an evaluation system which includes the evaluation device.
Fig. 3 is a table representing an example of care evaluation information.
Fig. 4 is a representation of an example of non-practice evaluation information.
Fig. 5 is representation of another example of the non-practice evaluation information.
Fig. 6 is a representation of an example of an evaluation method in an evaluation section.
Fig. 7 is a representation of another example of the evaluation method in the evaluation section.
Fig. 8 is a representation of an example of measure information.
Fig. 9 is a representation of an example of a method for displaying an evaluation result.
Fig. 10 is a flowchart illustrating example processing in the evaluation device.

### Description of Embodiments

### (Embodiment 1)

### [Outline of evaluation system]

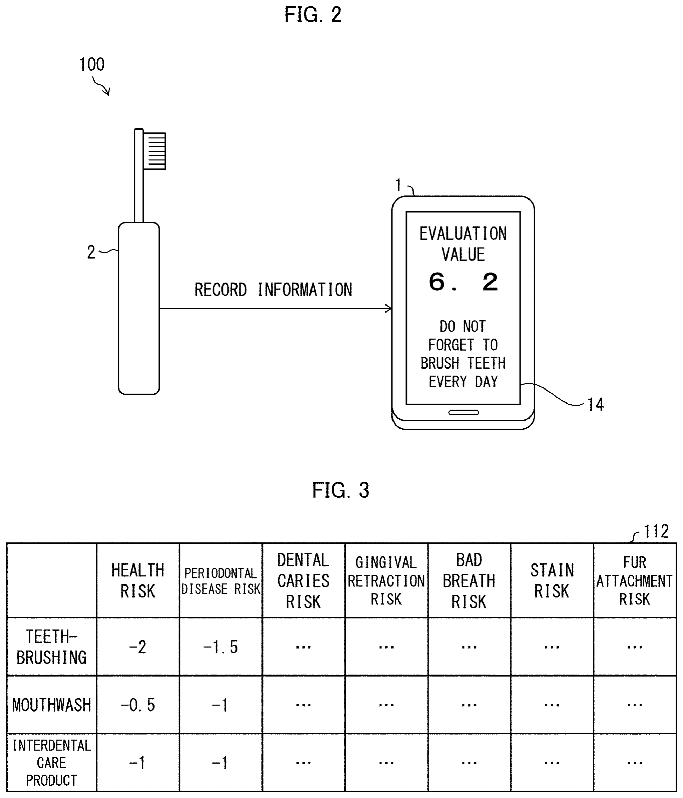

The configuration of an evaluation system 100 in accordance with an embodiment of the present invention is described here on the basis of Fig. 2. Fig. 2 is a diagram illustrating an example configuration of the evaluation system 100, which includes an evaluation device 1. The evaluation system 100 has the function of properly evaluating the oral state of a subject, in consideration of the length of an oral care non-practice period. Examples of the oral care herein include teeth-brushing (also including the brushing with use of a specific dentifrice), use of a mouthwash, use of an interdental care product, and scaling in a dental clinic.

In the evaluation system 100, the oral state of the subject is evaluated by quantifying, into numerical values, risks concerning the oral health state of the subject. Examples of the risks concerning the oral health state of a subject include health risk concerning an oral state, periodontal disease risk, dental caries risk, gingival retraction risk, bad breath risk, stain risk, and fur attachment risk, and the evaluation system 100 carries out evaluation for each of these risks. Note that the health risk concerning an oral state is a comprehensive risk index which indicates a reduction in the health level of an oral cavity, which leads to tooth loss.

### (Correspondence between risk and time passage of oral care non-practice period)

In the logarithmic phase, in which bacteria involved in periodontal disease or the like grow, the bacteria grow exponentially. For example, approximately 8 hours after bacteria attach to pellicle of salivary glycoproteins formed on enamel, a microcolony is formed, and plaque is formed accordingly. In such formation, maturation of the plaque with time passage results in significant growth of the bacteria.

Specifically, in the initial colonization phase, in which plaque is formed, cocci, aerobic *Nocardia,* and *Neisseria* grow, and in the proliferation phase, which is 48 hours after the plaque formation, facultative anaerobic bacteria, such as *Actinomyces* and *Corynebacterium,* increases while the bacteria in the colonization phase decrease. Further, when left, without any oral care, for 3 to 5 days from the plaque formation, *Veillonella, Fusobacterium* (obligate anaerobic bacteria), and *Spirochaeta* (motile organism) increase. Bacteria which grow in an oral cavity have various influences on an oral health state. In particular, bacteria of *Fusobacterium, Spirochaeta,* etc., which grow in the latter half of the plaque formation, are directly or indirectly involved in destruction of periodontal tissue.

Moreover, when starting to harden into tartar, plaque is difficult to remove via common teeth-brushing. In regard to the site in which hardening into tartar has started, however high the result of evaluation of a single time of brushing, there is a high possibility that hardening into tartar will proceed and an oral health state will worsen accordingly. Plaque starts to harden into tartar after a lapse of one or two days from plaque formation, whereas it takes 12 days on average for the plaque to completely harden into tartar. However, it is considered most likely that tartar is formed in a site which is always left unbrushed.

As described above, there is a cause-and-effect relationship between time passage of an oral care non-practice period and worsening of an oral health state. Further, in order to maintain a good oral health state, it is significantly important to inhibit the growth of bacteria to a minimum and carry out some procedure before tartar is completely formed. As such, in the present invention, by properly evaluating the oral state of a subject in consideration of time passage of an oral care non-practice period and presenting the evaluation to the subject, the subject is made aware of risks concerning the oral state, and the subject is prompted to carry out some procedure before the oral state worsens.

### [Configuration of evaluation system]

As illustrated in Fig. 2, the evaluation system 100 includes an evaluation device 1 and an electric toothbrush 2. When, for example, a brush portion of the electric toothbrush 2 is vibrated at a preset period and the subject places the electric toothbrush 2 on the teeth, the electric toothbrush 2 automatically performs brushing. The electric toothbrush 2, which has a communication function, communicates with the evaluation device 1. In the evaluation system 100, a manual toothbrush may be used instead of the electric toothbrush 2. In that case, the manual toothbrush may include a communication device.

### [Configuration of evaluation device]

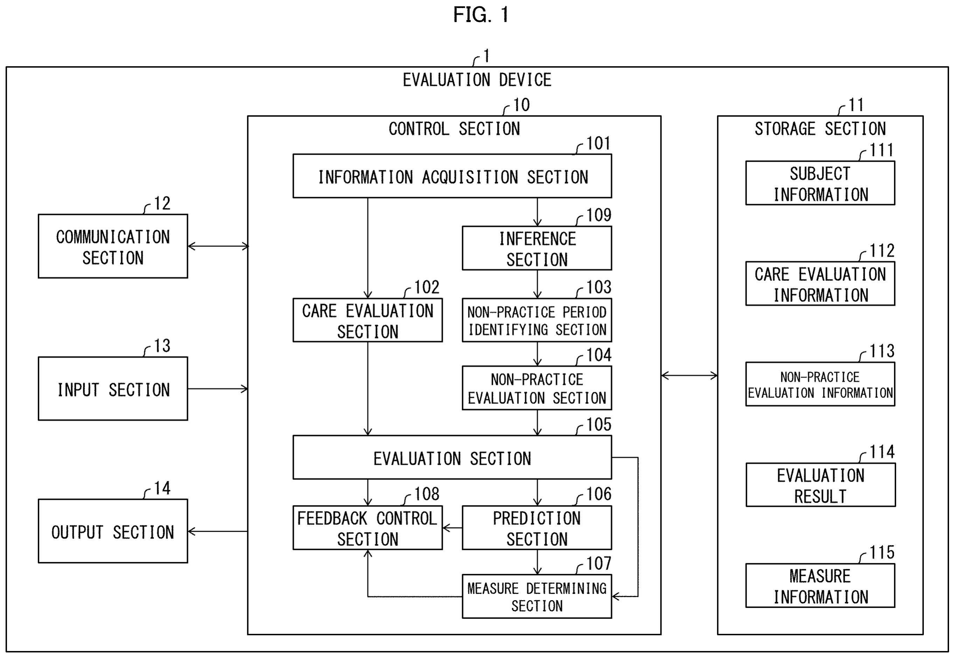

Fig. 1 is a block diagram illustrating an example configuration of the main part of the evaluation device 1 in accordance with an embodiment of the present invention. The evaluation device 1 identifies, on the basis of record information which includes information on oral care practiced, the length of a non-practice period, which is a period during which a subject of oral state evaluation does not practice predetermined oral care, and evaluates the oral state of the subject depending on the length of the non-practice period. The evaluation device 1 may be, for example, an information processing terminal owned by the subject. As illustrated in Fig. 1, the evaluation device 1 includes a control section 10, a storage section 11, a communication section 12, an input section 13, and an output section 14.

The control section 10 performs overall control of the sections of the evaluation device 1. As an example, the control section 10 may be a central processing unit (CPU). The control section 10 retrieves a control program, which is software stored in the storage section 11, and loads the control program into a memory such as a random access memory (RAM) to execute various functions. The configuration of the control section 10 will be described later in detail.

The storage section 11 (record accumulation device) stores various kinds of data to be used by the evaluation device 1. The storage section 11 has stored therein, in particular, subject information 111, care evaluation information 112, non-practice evaluation information 113, evaluation result 114, and measure information 115. The above pieces of information will be described later in detail. The communication section 12 communicates with the electric toothbrush 2.

In the present embodiment, the storage section 11 functions as the record accumulation device. However, a record accumulation device which accumulates record information is not limited thereto, and may be provided so as to be separate from the evaluation device 1. In that case, the record accumulation device may be provided in, for example, the electric toothbrush 2. Further, in a case where a manual toothbrush is used instead of the electric toothbrush 2 in the evaluation system 100, the manual toothbrush may be provided with the record accumulation device. Further, record information of the electric toothbrush 2 or the manual toothbrush which does not have a communication function may be directly inputted by the subject to the evaluation device 1, and an information acquisition section 101 may acquire the record information.

In a case where the record information is directly inputted by the subject to the evaluation device 1, the evaluation device 1 may have the function for preventing an omission of an input of oral care practiced. For example, the evaluation device 1 may be provided with an application via which a popup confirming whether interdental care is practiced and whether a mouthwash is used is displayed on the output section 14 upon the expiration of a brushing timer, which is used as a timer for teeth-brushing. This makes it possible to make sure to input oral care practiced.

The input section 13 receives inputs of the various kinds of data to the evaluation device 1. The input section 13 receives, for example, the subject information 111 inputted by the subject. For example, the subject information 111 includes various kinds of information regarding salivary constituents, the amount of saliva, meal timing, the details of meal, a frequency at which to open a mouth, a frequency at which to clench the teeth, dental occlusion, a tongue, chewing, drug-taking information, physical activities, the amount of activity, general health state (with or without disease, blood sugar level, blood pressure, skeletal muscle mass, etc.), and sleep. The subject information 111 may also include participation factors associated with, for example, how readily the risks concerning an oral health state increase. Examples of the participation factors include dentition information (teeth arrangement, prostheses, loss, periodontal pocket depth, etc.), buccal mucosa and dental arch (personal characteristics which lead to difficulty in brushing), and the softness, thinness, and presence or absence of swelling of gums.

The input section 13 also receives information on oral care (e.g., practice of interdental care and use of a mouthwash) practiced by the subject other than teeth-brushing. The input of oral care other than teeth-brushing may be performed by the subject together with the date and time when the oral care was practiced.

The output section 14 (presentation device) presents, to the subject, feedback information depending on an evaluation result acquired from a feedback control section 108, which will be described later. The output section 14 is, for example, a liquid crystal display. The output section 14 may include the function of outputting sound or vibration. The output section 14 need not be included in the evaluation device 1, and may be in such a form as to be provided outside the evaluation device 1 and connected to the evaluation device 1.

### (Configuration of control section)

The control section 10 includes an information acquisition section 101, a care evaluation section 102, a non-practice period identifying section 103, a non-practice evaluation section 104, an evaluation section 105, a prediction section 106, a measure determining section 107, a feedback control section 108, and an inference section 109. In Embodiment 1, the inference section 109 is not essential. The inference section 109 will be described in detail in Embodiment 2.

### (Information acquisition section)

The information acquisition section 101 acquires various kinds of data necessary for evaluation of an oral state. These pieces of data include record information indicative of a record of use of the electric toothbrush 2, and subject information 111. The information acquisition section 101 accumulates record information in the storage section 11. The record information includes, for example, information on the date and time at which the subject practiced teeth-brushing, information on the acceleration of the brushing, and brushing pressure. Further, the record information also includes oral care (e.g., practice of interdental care and use of a mouthwash) practiced by the subject other than teeth-brushing.

### (Care evaluation section)

The care evaluation section 102 calculates, depending on the details of the oral care practiced by the subject, a care evaluation value indicative of an influence which practice of oral care by the subject had on the oral state during a target period the oral state during which is to be evaluated. The care evaluation section 102 identifies oral care practiced in the target period and calculates a care evaluation value on the basis of the oral care identified with reference to the care evaluation information 112. The target period may be set by the subject in advance.

Although time passage of an oral care non-practice period increases risks to the health state inside an oral cavity, the risks are mitigated when oral care is provided. Fig. 3 is a table illustrating an example of the care evaluation information 112. In the care evaluation information 112, oral care practiced is associated with a mitigated risk for each of the risks concerning an oral health state, as illustrated in Fig. 3. The mitigated risk is quantification, into numerical value, of the risk mitigated by oral care practiced. For example, the care evaluation information 112 is preset with use of an empirical value or the like. Further, in "···" of the care evaluation information 112 indicated in Fig. 3, a value determined with use of the empirical value or the like is inputted.

The care evaluation section 102 calculates, in accordance with the care evaluation information 112, a care evaluation value on the basis of oral care practiced. For example, in a case where the subject practices teeth-brushing as oral care, the care evaluation section 102 calculates the care evaluation value as "-2".

The oral care practiced by the subject in a case where the care evaluation section 102 calculates the care evaluation value is not limited to teeth-brushing, use of a mouthwash, or use of an interdental care product, which are indicated in Fig. 3. Said oral care may include tongue scraping, which is effective in removing the fur of the tongue, a specific care brush, and scaling in a dental clinic. Further, the care evaluation value may be calculated depending on the way to brush the teeth, the amount of teeth-brushing time, and the type, configuration, and constituents of an oral care product used. Moreover, the care evaluation value may be corrected on the basis of an actual participation factor (such as a periodontal pocket depth).

### (Non-practice period identifying section)

Risks concerning an oral health state increase mainly due to time passage of an oral care non-practice period. Specifically, for example, the health risk concerning an oral state is increased by growth of bacteria, hardening of plaque into tartar, and an increase in bacteria causing periodontal disease or bacteria causing dental caries that are associated with time passage of the oral care non-practice period. The periodontal disease risk increases due to an increase in bacteria causing periodontal disease and progression of periodontal disease that are associated time passage of the oral care non-practice period. The dental caries risk is increased by growth of bacteria, hardening of plaque into tartar, and an increase in bacteria causing dental caries that are associated with time passage of the oral care non-practice period. The gingival retraction risk is increased by progression of periodontal disease associated with time passage of the oral care non-practice period. The bad breath risk increases due to growth of bacteria and an increase in bacteria causing bad breath that are associated with time passage of the oral care non-practice period. The stain risk increases due to growth of bacteria, hardening of plaque into tartar, and colonization of the stain constituents on teeth that are associated with time passage of the oral care non-practice period. The fur attachment risk increases due to growth of bacteria associated with time passage of the oral care non-practice period.

As such, the non-practice period identifying section 103 identifies the length of the non-practice period, which is a period during which a subject of oral state evaluation does not practice predetermined oral care in the target period. The non-practice period identifying section 103 may identify the length of the non-practice period with respect to each of the plurality of areas into which an oral cavity of the subject is divided.

In a case where the non-practice period identifying section 103 identifies the length of the non-practice period with respect to each of the plurality of areas into which the oral cavity of the subject is divided, an area containing a plurality of teeth may be set as a unit area, or an individual tooth or a portion (such as an interdental portion or a portion near the gingiva) of the individual tooth may be set as a unit area. Further, in the non-practice period identifying section 103, an area which includes mucosa and a tongue may be set as a unit area.

### (Non-practice evaluation section)

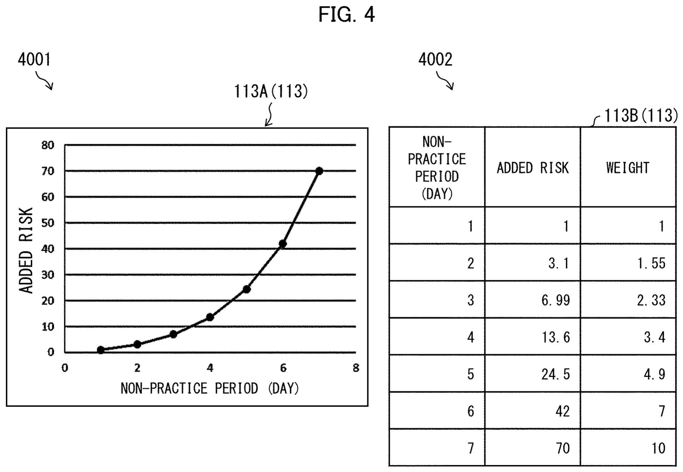

The non-practice evaluation section 104 calculates a non-practice evaluation value indicative of an influence which the non-practice period in the target period had on the oral state. Fig. 4 is a diagram illustrating an example of non-practice evaluation information 113, and the diagram illustrates an example of the correspondence between the non-practice period and the occurrence of an arbitrary risk (e.g., health risk concerning an oral state) among the risks concerning an oral health state. The reference sign 4001 of Fig. 4 indicates non-practice evaluation information 113A, in which the non-practice evaluation information 113 is represented in a graph, and the reference sign 4002 of Fig. 4 indicates non-practice evaluation information 113B, in which the non-practice evaluation information 113 is represented in a table. The non-practice evaluation information 113 is present for each of the risks concerning an oral health state to be evaluated.

As illustrated in 4001 of Fig. 4, and 4002 of Fig. 4, in the non-practice evaluation information 113, the oral care non-practice period is associated with the numerical value of an added risk. The added risk is quantification, into numerical value, of the risk increased by the oral care non-practice period. The non-practice evaluation information 113 is preset, for example, on the basis of the correspondence between time passage and the growth of bacteria in the proliferation phase. The "weight" indicated in the non-practice evaluation information 113B is a numerical value obtained by dividing the added risk by the non-practice period (day).

In accordance with the non-practice evaluation information 113, the non-practice evaluation section 104 calculates a non-practice evaluation value of the arbitrary risk on the basis of the oral care non-practice period. For example, in a case where the time passage of the non-practice period identified in the non-practice period identifying section 103 is a lapse of 2 days, the non-practice evaluation section 104 refers to the non-practice evaluation information 113B and calculates the non-practice evaluation value as "3.1".

### (Another example of calculating non-practice evaluation value)

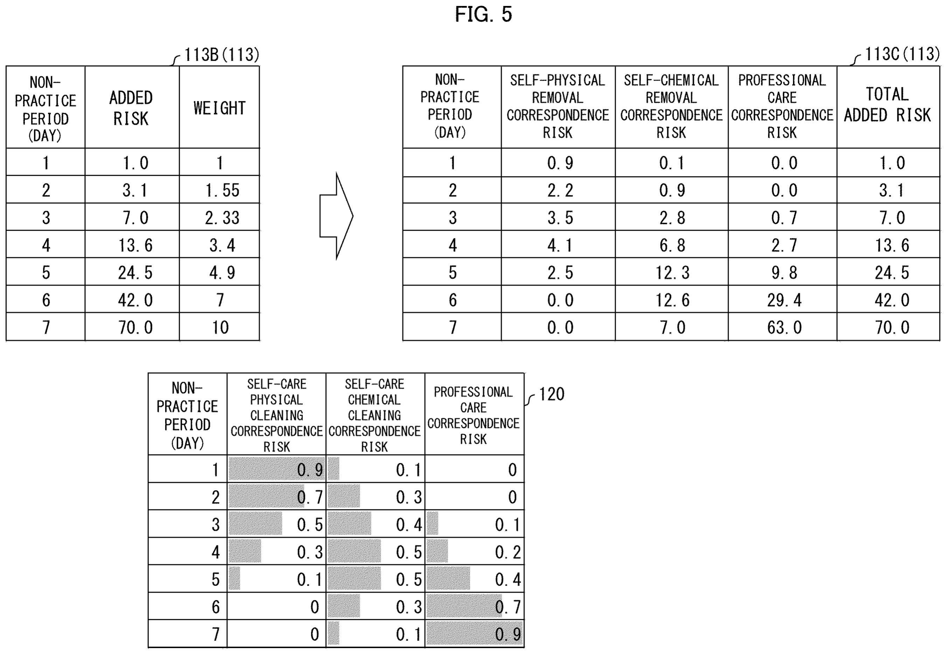

Fig. 5 is a diagram illustrating another example of the non-practice evaluation information 113. The non-practice evaluation section 104 may calculate a non-practice evaluation value with reference to non-practice evaluation information 113C, in which the added risk for the arbitrary risk of the non-practice evaluation information 113B is divided into a self-physical removal correspondence risk, a self-chemical removal correspondence risk, and a professional care correspondence risk.

The self-physical removal correspondence risk indicates the added risk caused when the physical cleaning, such as teeth-brushing or interdental brushing, is not practiced. The self-chemical removal correspondence risk indicates the added risk caused when the chemical cleaning performed with use of a mouthwash or a dentifrice is not practiced. The professional care correspondence risk indicates the added risk caused when scaling or the like in a dental clinic is not practiced.

The non-practice evaluation information 113C is formed on the basis of risk breakdown information 120. The risk breakdown information 120 is information in which the respective proportions of the self-physical removal correspondence risk, the self-chemical removal correspondence risk, and the professional care correspondence risk are set in each unit non-practice period.

The non-practice evaluation information 113C multiplies the proportion of each risk (self-physical removal correspondence risk, self-chemical removal correspondence risk, and professional care correspondence risk) set on a unit non-practice period basis in the risk breakdown information 120, by the added risk corresponding to each unit non-practice period in the non-practice evaluation information 113B, to obtain an added risk for each risk in each unit non-practice period of the arbitrary risk.

For example, in the risk breakdown information 120, on the 3rd day of the non-practice period, self-physical removal correspondence risk:self-chemical removal correspondence risk:professional care correspondence risk is set at 0.5:0.4:0.1. Since the added risk on the 3rd day of the non-practice period in the non-practice evaluation information 113B is 7.0, the added risks for each risk on the 3rd day of the non-practice period in the non-practice evaluation information 113C are 0.5 x 7.0 = 3.5, 0.4 x 7.0 = 2.8, and 0.1 x 7.0 = 0.7 in the order of the self-physical removal correspondence risk, the self-chemical removal correspondence risk, and the professional care correspondence risk.

As described above, the non-practice evaluation section 104 may calculate a non-practice evaluation value on the basis of the non-practice evaluation information 113C. For example, in a case where, on the 2nd day of the target period, the non-practice period of a self-care physical cleaning identified by the non-practice period identifying section 103 is one day long and the non-practice period of a self-care chemical cleaning and the professional care correspondence risk is two days long, the non-practice evaluation value may be calculated as follows. Specifically, the non-practice evaluation section 104 may refer to the non-practice evaluation information 113C to calculate the non-practice evaluation value of the self-care physical cleaning as "0.9", calculate the non-practice evaluation value of the self-chemical removal correspondence risk as "0.9", calculate the non-practice evaluation value of the professional care correspondence risk as "0.0", and thus calculate the non-practice evaluation value of the 2nd day in the target period as 1.8.

Further, in a case where the non-practice evaluation section 104 refers to the non-practice evaluation information 113C, the care evaluation section 102 may also carry out evaluation with the division into the self-physical removal correspondence risk, the self-chemical removal correspondence risk, and the professional care correspondence risk.

For example, in a case of practicing oral care in which teeth-brushing with a toothbrush alone is practiced for 3 minutes, the care evaluation section 102 may calculate the care evaluation value by which the total value of the self-physical removal correspondence risk at the time point at which the oral care is practiced is reduced by 60%. Further, in a case where oral care in which teeth-brushing with a toothbrush with a dentifrice is practiced for 3 minutes, the care evaluation section 102 may calculate the care evaluation value by which the total value of the self-physical removal correspondence risk at the time point at which the oral care is practiced is reduced by 60% and the self-chemical removal correspondence risk at said time point is reduced by 30%. Moreover, in a case where oral care in which scaling for 3 minutes in a dental clinic is practiced, the care evaluation section 102 may calculate the care evaluation value by which the total value of the self-physical removal correspondence risk at the time point at which oral care is practiced is reduced by 70%, the self-chemical removal correspondence risk at said time point is reduced by 70%, and the total value of the professional care correspondence risk at said time point is reduced by 70%.

### (Evaluation section)

The evaluation section 105 evaluates the oral state of the subject depending on the length of the non-practice period.

Specifically, the evaluation section 105 uses a care evaluation value and a non-practice evaluation value to calculate an evaluation value indicative of a result of evaluation of the oral state of the subject. This makes it possible to calculate a reasonable evaluation value in consideration of each of the influence which practice of oral care had on the oral state and the influence which a non-practice period had on the oral state.

The evaluation section 105 evaluates each of a plurality of types of risks concerning the oral state. This makes it possible to evaluate the degrees of the respective types of risks. The evaluation section 105 evaluates each of the risks which are, for example, health risk concerning an oral state, periodontal disease risk, dental caries risk, gingival retraction risk, bad breath risk, stain risk, and fur attachment risk. According to the present embodiment, the evaluation section 105 sets the minimum evaluation value at 0. The evaluation value being higher indicates a state of an increasing risk, and the evaluation value being lower indicates that an oral state is successfully kept healthy.

The evaluation section 105 may carry out evaluation depending on the length of the non-practice period, for each of a plurality of areas for which the lengths of the non-practice period are identified by the non-practice period identifying section 103. This makes it possible to make a subject aware of an area that continuously remains without oral care in the oral cavity.

Further, the evaluation section 105 may evaluate the oral state of a subject on the basis of subject information regarding the subject. For example, the evaluation value calculated by the evaluation section 105 may be corrected on the basis of the state of a participation factor (such as a periodontal pocket depth). This makes it possible to calculate a reasonable evaluation value in consideration of a subject-specific individual difference.

Further, the evaluation section 105 may evaluate the oral state of a subject on the basis of behavioral data on the subject. For example, the evaluation value calculated by the evaluation section 105 may be corrected on the basis of a plurality of pieces of behavioral data. For example, for the health risk concerning an oral state and the bad breath risk, the evaluation value may be corrected on the basis of the amount of teeth-brushing time, and for the periodontal disease risk, the evaluation value may be corrected on the basis of the amount of teeth-brushing time and the position or angle of a toothbrush on a tooth surface. For the dental caries risk, the evaluation value may be corrected on the basis of the amount of teeth-brushing time, the date and time of inoculation of carbohydrate, and the amount of carbohydrate intake, and for gingival retraction risk, the evaluation value may be corrected on the basis of the amount of teeth-brushing time and brushing pressure. For the stain risk, the evaluation value may be corrected on the basis of the amount of teeth-brushing time and the date and time of intake of the cause of stain, and for the fur attachment risk, the evaluation value may be corrected on the basis of the date and time and the amount of time of use of a tongue scraper.

Further, in a case where the non-practice evaluation section 104 refers to the non-practice evaluation information 113C, the evaluation section 105 may also carry out evaluation with the division into the self-physical removal correspondence risk, the self-chemical removal correspondence risk, and the professional care correspondence risk.

The evaluation section 105 stores the evaluation value evaluated in the storage section 11 as an evaluation result 114.

### (Example of evaluation method in evaluation section)

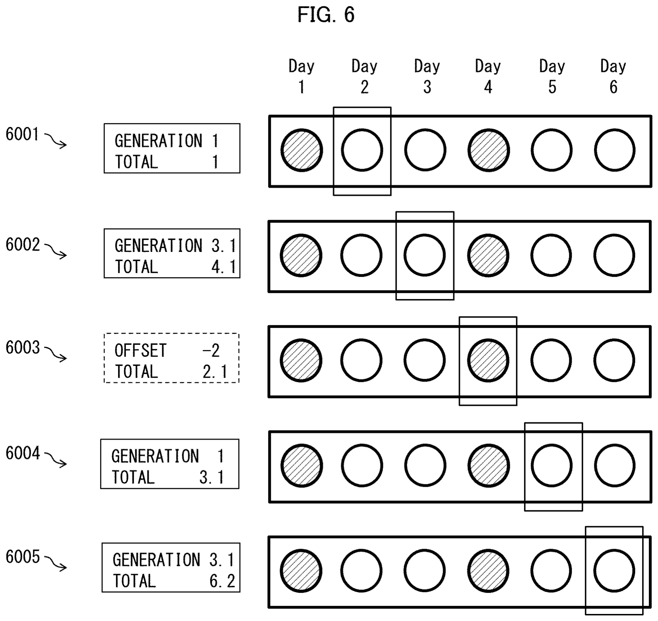

Fig. 6 is a diagram illustrating an example of the evaluation method in the evaluation section 105. On the basis of Fig. 6, the evaluation method carried out by the evaluation section 105 with respect to the target period from Day 1 to Day 6 is described here. The reference sign 6001 of Fig. 6 is a diagram illustrating the evaluation value for Day 2, the reference sign 6002 of Fig. 6 is a diagram illustrating the evaluation value for Day 3, the reference sign 6003 of Fig. 6 is a diagram illustrating the evaluation value for Day 4, the reference sign 6004 of Fig. 6 is a diagram illustrating the evaluation value for Day 5, and the reference sign 6005 of Fig. 6 is a diagram illustrating the evaluation value for Day 6.

In Fig. 6, the hatched circle indicates that an oral care was practiced, and the white circle indicates that oral care was not practiced. That is, among the days from Day 1 to Day 6 of the target period, a subject practiced oral care on Day 1 and Day 4, and the subject did not practice oral care on Day 2, Day 3, Day 5 and Day 6. Assume that on Day 1 and Day 4, oral care (e.g., teeth-brushing for 3 minutes) with a toothbrush was performed. In the following description, for ease of description, an evaluation method carried out by the evaluation section 105 regarding the health risk concerning an oral state is described below, and the non-practice evaluation information 113B indicates the correspondence between the occurrence of a health risk concerning an oral state and time passage.

The evaluation section 105 sets a unit non-practice period and a target period. For example, the unit non-practice period and the target period are arbitrarily set by the subject. In the example illustrated in Fig. 6, the unit non-practice period is set to 1 day, and the target period is set to from Day 1 to Day 6.

The evaluation section 105 determines the initial value of the risk. In the example illustrated in Fig. 6, the initial value of the risk is set at "0". The initial value of the risk is not limited thereto, but may be set on the basis of past records.

Oral care by teeth-brushing was practiced on Day 1, and the care evaluation section 102 calculates the care evaluation value as "-2" with reference to the care evaluation information 112 (Fig. 3). However, since the initial value is "0", which is the minimum value of the evaluation value, the total value of the care evaluation value and the non-practice evaluation value on Day 1 is calculated as "0".

As illustrated in 6001 of Fig. 6, since oral care was not practiced on Day 2, the non-practice period identifying section 103 identifies the length of the non-practice period for Day 2 as 1 day. The non-practice evaluation section 104 refers to the non-practice evaluation information 113B (Fig. 4), and calculates the non-practice evaluation value as "1". In other words, the value "1" is generated as the risk on Day 2. The evaluation section 105 adds the non-practice evaluation value "1" to the evaluation value "0" for Day 1, and calculates the total value (evaluation value) of the care evaluation value and the non-practice evaluation value for Day 2 as "1".

As illustrated in 6002 in Fig. 6, since continuing from Day 2, oral care was not practiced on Day 3, the non-practice period identifying section 103 identifies the length of the non-practice period for Day 3 as 2 days. The non-practice evaluation section 104 refers to the non-practice evaluation information 113B (Fig. 4), and calculates the non-practice evaluation value as "3.1". The evaluation section 105 adds the non-practice evaluation value "3.1" to the evaluation value "1" for Day 2, and calculates the total value of the care evaluation value and the non-practice evaluation value for Day 3 as "4.1".

As illustrated in 6003 of Fig. 6, oral care by teeth-brushing is practiced on Day 4, and the care evaluation section 102 calculates the care evaluation value as "-2" with reference to the care evaluation information 112 (Fig. 3). In other words, the risk is subjected to an offset of "-2" on Day 4. The evaluation section 105 adds the care evaluation value "-2" to the evaluation value "4.1" for Day 3, and calculates the total value of the care evaluation value and non-practice evaluation value for Day 4 as "2.1".

As illustrated in 6004 of Fig. 6, since oral care was practiced on Day 4 and oral care was not practiced on Day 5, the non-practice period identifying section 103 identifies the length of the non-practice period for Day 5 as 1 day. The non-practice evaluation section 104 refers to the non-practice evaluation information 113B (Fig. 4), and calculates the non-practice evaluation value as "1". The evaluation section 105 adds the non-practice evaluation value "1" to the evaluation value "2.1" for Day 4, and calculates the total value of the care evaluation value and the non-practice evaluation value for Day 5 as "3.1".

As illustrated in 6005 of Fig. 6, since continuing form Day 5, oral care was not practiced on Day 6, the non-practice period identifying section 103 identifies the length of the non-practice period for Day 6 as 2 days. The non-practice evaluation section 104 refers to the non-practice evaluation information 113B (Fig. 4), and calculates the non-practice evaluation value as "3.1". The evaluation section 105 adds the non-practice evaluation value "3.1" to the evaluation value "3.1" for Day 5, and calculates the total value of the care evaluation value and the non-practice evaluation value for Day 6 as "6.2". The evaluation section 105 evaluates the evaluation value of the health risk concerning an oral state for this target period as "6.2".

As described above, the evaluation section 105 calculates the evaluation value by setting a unit non-practice period and a target period with respect to a plurality of types of risks concerning an oral health state and calculating the total value of the care evaluation value and the non-practice evaluation value on a unit non-practice period basis.

### (Another example of evaluation method in evaluation section)

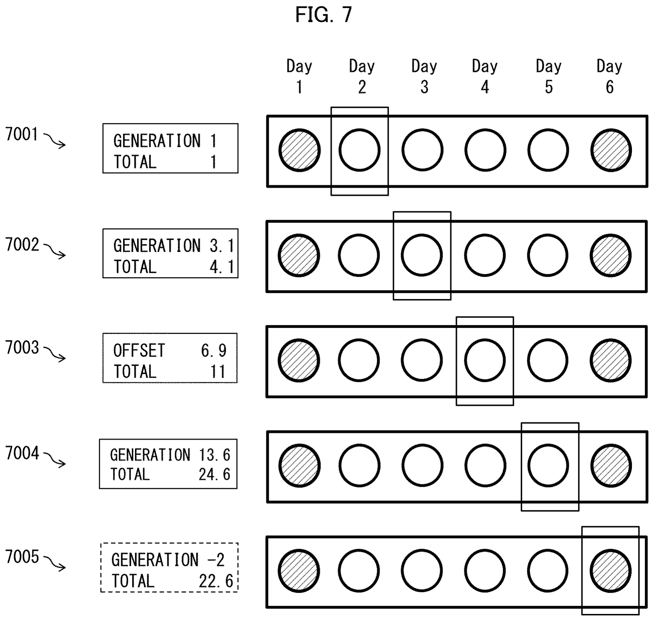

Fig. 7 is a diagram illustrating another example of the evaluation method in the evaluation section 105. On the basis of Fig. 7, another example of the evaluation method in the evaluation section 105 is described. The reference sign 7001 of the drawing is a diagram illustrating the evaluation value for Day 2, the reference sign 7002 of Fig. 7 is a diagram illustrating the evaluation value for Day 3, the reference sign 7003 of Fig. 7 is a diagram illustrating the evaluation value for Day 4, the reference sign 7004 of Fig. 7 is a diagram illustrating the evaluation value for Day 5, and the reference sign 7005 of Fig. 7 is a diagram illustrating the evaluation value for Day 6.

In Fig. 7, the hatched circle indicates that an oral care was practiced, and the white circle indicates that oral care was not practiced. That is, among the days from Day 1 to Day 6 of the target period, a subject practiced oral care on Day 1 and Day 6, and the subject did not practice oral care from Day 2 to Day 5. Assume that on Day 1 and Day 6, oral care (e.g., teeth-brushing for 3 minutes) with a toothbrush was performed. In the following description, for ease of description, an evaluation method regarding the health risk concerning an oral state is described below, and the non-practice evaluation information 113B indicates the correspondence between the occurrence of a health risk concerning an oral state and time passage. In the example illustrated in Fig. 7, the unit non-practice period is set as 1 day, the target period is set to from Day 1 to Day 6, and the initial value of the risk is set as "0".

Oral care by teeth-brushing is practiced on Day 1, and the care evaluation section 102 calculates the care evaluation value as "-2" with reference to the care evaluation information 112 (Fig. 3). However, since the initial value is "0", which is the minimum value of the evaluation value, the total value of the care evaluation value and the non-practice evaluation value for Day 1 is "0".

As illustrated in 7001 of Fig. 7, since oral care was not practiced on Day 2, the non-practice period identifying section 103 identifies the length of the non-practice period for Day 2 as 1 day. The non-practice evaluation section 104 refers to the non-practice evaluation information 113B (Fig. 4), and calculates the non-practice evaluation value as "1". The evaluation section 105 adds the non-practice evaluation value "1" to the evaluation value "0" for Day 1, and calculates the total value of the care evaluation value and the non-practice evaluation value for Day 2 as "1".

As illustrated in 7002 for Fig. 7, since continuing from Day 2, oral care was not practiced on Day 3, the non-practice period identifying section 103 identifies the length of the non-practice period for Day 3 as 2 days. The non-practice evaluation section 104 refers to the non-practice evaluation information 113B (Fig. 4), and calculates the non-practice evaluation value as "3.1". The evaluation section 105 adds the non-practice evaluation value "3.1" to the evaluation value "1" for Day 2, and calculates the total value of the care evaluation value and the non-practice evaluation value for Day 3 as "4.1".

As illustrated in 7003 of Fig. 7, since oral care was not practiced on Day 4, consecutively from Day 2 to Day 4, the non-practice period identifying section 103 identifies the length of the non-practice period for Day 4 as 3 days. The non-practice evaluation section 104 refers to the non-practice evaluation information 113B (Fig. 4), and the non-practice evaluation value is calculated as "6.9". According to the non-practice evaluation information 113B of Fig. 4, the added risk for the 3rd day of the non-practice period is 6.99. However, for ease of description, the value "6.9" obtained by rounding down to the first decimal place is adopted. The evaluation section 105 adds the non-practice evaluation value "6.9" to the evaluation value "4.1" for Day 3, and calculates the total value of the care evaluation value and the non-practice evaluation value for Day 4 as "11".

As illustrated in 7004 of Fig. 7, since oral care was not practiced on Day 5, consecutively from Day 2 to Day 5, the non-practice period identifying section 103 identifies the length of the non-practice period for Day 5 as 4 days. The non-practice evaluation section 104 refers to the non-practice evaluation information 113B (Fig. 4), and the non-practice evaluation value is calculated as "13.6". The evaluation section 105 adds the non-practice evaluation value "13.6" to the evaluation value "11" for Day 4, and calculates the total value of the care evaluation value and the non-practice evaluation value for Day 5 as "24.6".

As illustrated in 7005 of Fig. 7, oral care by teeth-brushing is practiced on Day 6, and the care evaluation section 102 calculates the care evaluation value as "-2" with reference to the care evaluation information 112 (Fig. 3). The evaluation section 105 adds the care evaluation value "-2" to the evaluation value "24.6" for Day 5, and calculates the total value of the care evaluation value and the non-practice evaluation value for Day 6 as "22.6". The evaluation section 105 evaluates the evaluation value for this target period as "22.6".

### (Prediction section)

The prediction section 106 predicts, on the basis of the time-series result of the evaluation, the result of evaluation at a time point after the time point when said evaluation was carried out. This makes it possible to prompt a subject to take an action in anticipation of a future oral state.

In regard to the prediction of a result of evaluation, a future oral state may be predicted by, for example, predicting an oral care practice day, on which oral care is considered to be practiced in the future, on the basis of the tendency of the past oral care practice and calculating an evaluation value after a lapse of a predetermined period of time. Although it is not essential to include the prediction section 106 in the control section 10 in the present embodiment, it is preferable to include the prediction section 106 in the control section 10.

### (Measure determining section)

The measure determining section 107 determines a measure to improve a result of evaluation carried out by the evaluation section 105, the measure depending on the result of evaluation. This makes it possible to make a subject aware of the measure to improve a result of evaluation.

Examples of the measure to improve a result of evaluation are expected to include: using a specific oral care product, changing an operation setting of an electric toothbrush, changing the way to brush the teeth, changing the timing of teeth-brushing, changing the amount of teeth-brushing time, and visiting a dentist.

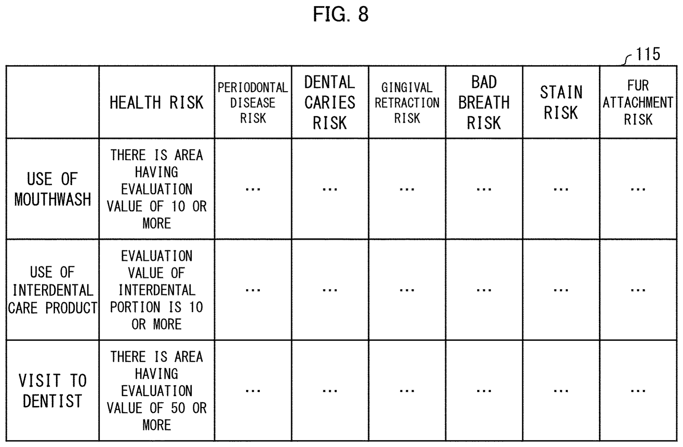

Fig. 8 is a table illustrating an example of the measure information 115, in which the correspondence between the risks concerning an oral health state and the measures. As illustrated in Fig. 8, in the measure information 115, the result of evaluation for each of the risks concerning an oral health state is associated with the measures. In measure information 115, for example, use of a mouthwash, use of an interdental care product, and a visit to a dentist are set as the measures.

The measure determining section 107 determines a measure for each of the risks concerning an oral health state, according to the measure information 115 and on the basis of the evaluation value for each risk calculated in the evaluation section 105. For example, in a case where there is an area having an evaluation value of 10 or more regarding the health risk concerning an oral state, the measure information 115 determines "use of a mouthwash" as a measure to improve the result of evaluation.

The measure determining section 107 may determine a measure against a plurality of types of risks concerning an oral health state, without limitation to the risk above. For example, the measure determining section 107 may determine a measure for eliminating a plurality of types of risks concerning an oral health state at once.

Further, the measure determining section 107 may determine the measure on the basis of the result of evaluation, predicted by the prediction section 106, of the future oral state of a subject. Further, the measure determining section 107 may have the function of granting a specific reward (e.g., points) to the subject, on the basis of the transition of the risks concerning an oral health state and depending on the amounts of reductions in the evaluation values. Although it is not essential to include the measure determining section 107 in the control section 10 in the present embodiment, it is preferable to include the measure determining section 107 in the control section 10.

### (Feedback control section)

By creating the feedback information depending on the result of evaluation of a subject and displaying said feedback information on the output section 14, the feedback control section 108 presents, to the subject, the feedback information regarding the evaluation of the risks concerning an oral health state. The evaluation result includes, for example,: the evaluation value calculated by the evaluation section 105; the result, predicted by the prediction section 106, of evaluation of the future oral state of the subject; and the measure determined by the measure determining section 107. The feedback control section 108 may inform the subject of the feedback information with sound or vibration.

### (Example of result display screen)

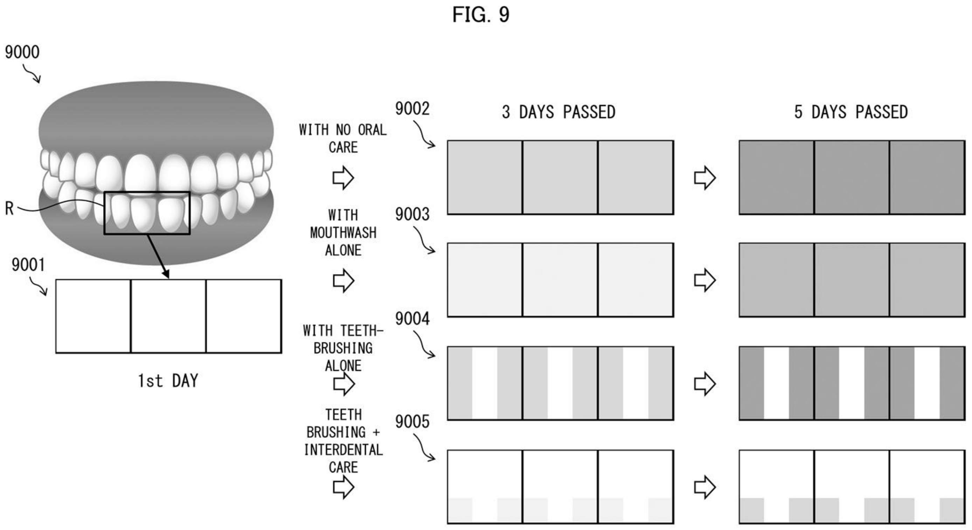

Fig. 9 is a diagram illustrating an example of a method for displaying the evaluation result. As illustrated in Fig. 9, the feedback control section 108 may present the feedback information to the subject by displaying risk levels on the output section 14 on a location-by-location basis in an illustration of a teeth model.

The reference sign 9001 of Fig. 9 is a diagram illustrating the initial state (1st day) in an area R of the teeth model indicated by 9000 of Fig. 9. The reference sign 9002 of Fig. 9 is a diagram illustrating an example of the display of the results of evaluation of the states in which 3 days have passed and 5 days have passed in the area R with no oral care practiced. The reference sign 9003 of Fig. 9 is a diagram illustrating an example of the display of the results of evaluation of the states in which 3 days have passed and 5 days have passed in the area R with oral care with a mouthwash alone practiced. The reference sign 9004 of Fig. 9 is a diagram illustrating an example of the display of the results of evaluation of the states in which 3 days have passed and 5 days have passed in the area R with oral care by teeth-brushing alone practiced. The reference sign 9005of Fig. 9 is a diagram illustrating an example of the display of the results of evaluation of the states in which 3 days have passed and 5 days have passed in the area R with oral care by teeth-brushing and interdental care practiced.

As illustrated in 9001 of Fig. 9 to 9005 of Fig. 9, the illustration of teeth may be displayed in a darker color when the risk is higher (the evaluation value is higher). As illustrated in 9004 of Fig. 9, a dark color at both ends of the illustration of teeth may express that continuous oral care by teeth-brushing alone increases risks to interdental places. Further, as illustrated in 9005 of Fig. 9, a light color at both ends of the illustration of teeth may express that interdental care mitigates the risks to interdental places.

Without limitation to the displays described above, the feedback control section 108 may directly display, on the output section 14, the numerical values regarding respective evaluation values of the risks concerning an oral health state, or the evaluation values may be summed up to be displayed on a time-series basis.

Further, the feedback control section 108 may replace the evaluation values with word by which the degrees of the evaluation values can be understood and display the words on the output section 14, or may express the degrees of the evaluation values by illustration in a display manner different from that illustrated in Fig. 9. As an example of the words by which the degrees of the evaluation values can be understood, if the evaluation value is 10 or more, the feedback control section 108 may display the words of "keep it up". As an examples of the illustration by which the degrees of the evaluation values can be understood, the feedback control section 108 may display a facial expression of a character, the amount of savings or debts, or the size of a bar graph, or may display some color change or the like in the illustration, as illustrated in Fig. 9.

The feedback control section 108 may report, to the subject, the degrees of the evaluation values with sound or vibration. Further, in a case where any of the plurality of types of risks concerning an oral state is greater than a predetermined threshold, the feedback control section 108 may display to that effect on the output section 14, and may report, to the subject with sound, for example.

Moreover, the feedback control section 108 may present a specific product depending on the measure determined in the measure determining section 107.

Further, by integrating the electric toothbrush 2 with the evaluation device 1, the feedback control section 108 may present the feedback information to the subject via the electric toothbrush 2. Although it is not essential to include the feedback control section 108 in the control section 10 in the present embodiment, it is preferable to include the feedback control section 108 in the control section 10.

As above, the evaluation device 1 includes: a non-practice period identifying section for identifying the length of a non-practice period, which is a period during which a subject of oral state evaluation does not practice predetermined oral care; and an evaluation section for evaluating the oral state of the subject depending on the length of the non-practice period. As such, the present invention makes it possible to properly evaluate an oral state, which has the characteristic of changing depending on the length of an oral care non-practice period.

Further, the evaluation system 100 includes: (1) a record accumulation device (storage section 11) for accumulating, with respect to one or more subjects of oral state evaluation, record information indicative of a record of use of a toothbrush (electric toothbrush 2) by said one or more subjects; (2) an evaluation device 1 for identifying, with respect to said one or more subjects, the length of a non-practice period, which is a period during which said one or more subjects do not practice predetermined oral care, on the basis of the record information, and evaluating an oral state depending on said length of the non-practice period; and (3) a presentation device (output section 14) for presenting feedback information depending on the result of evaluation. This makes it possible to receive the presentation of feedback information depending on a reasonable evaluation value in which the length of an oral care non-practice period is taken into consideration, and therefore makes it possible to prompt a subject to practice appropriate oral care and contribute to the maintenance and improvement of the oral state.

### (Flow of processing in evaluation device)

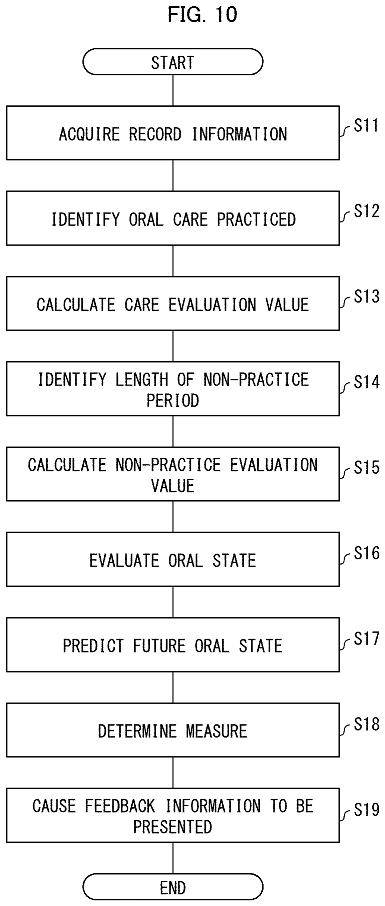

A flow of processing in the evaluation device 1 is described here on the basis of Fig. 10. Fig. 10 is a flowchart illustrating an example of the processing of the evaluation device 1.

In step S11, the information acquisition section 101 acquires record information indicative of a record of use of oral care practiced by the subject. In step S12, the care evaluation section 102 identifies the oral care practiced by the subject, and calculates (step S13) a care evaluation value with reference to the care evaluation information 112, depending on the oral care practiced by the subject.

In step S14 (non-practice period identifying step), the non-practice period identifying section 103 identifies the length of a non-practice period, which is a period during which the subject does not practice predetermined oral care in the target period. In step S15, the non-practice evaluation section 104 calculates a non-practice evaluation value based on the non-practice period identified by the non-practice period identifying section 103 and the non-practice evaluation information 113.

In step S16 (evaluation step), the evaluation section 105 evaluates the oral state of the subject. The evaluation section 105 evaluates the oral state of the subject by calculating an evaluation value with use of the care evaluation value and the non-practice evaluation value.

In step S17, the prediction section 106 predicts a future oral state on the basis of a time-series result of oral care evaluation. The prediction section 106 predicts, for example, an evaluation value of the oral state of the subject after a lapse of a predetermined period of time. In step S18, a measure is determined on the basis of the evaluation value of the evaluation carried out by the evaluation section 105 and the evaluation value of the future oral state predicted by the prediction section 106.

In step S19, the feedback control section 108 creates feedback information based on the results of step S16 to step S17, and causes the feedback information to be presented to the subject.

The functions of the evaluation device 1 can be implemented by a system constituted by a plurality of information processing devices, and the devices constituting the evaluation system 100 may be changed as appropriate. As such, the sections carrying out the steps illustrated in Fig. 10 are not limited to those described above. That is, the steps illustrated in Fig. 10 can be carried out by a single information processing device, or can be carried out by the plurality of information processing devices which share the processes of said steps thereamong.

As described above, the evaluation method of the present embodiment is (1) an evaluation method carried out by one or more information processing devices, the evaluation method including: a non-practice period identifying step (step S14) for identifying the length of a non-practice period, which is a period during which a subject of oral state evaluation does not practice predetermined oral care; and (2) an evaluation step (step S16) for calculating an evaluation value of the oral state of the subject depending on the length of the non-practice period. As such, the present invention makes it possible to properly evaluate an oral state, which has the characteristic of changing depending on the length of an oral care non-practice period.

### (Gingival retraction risk)

Among the risks concerning an oral health state, the health risk concerning an oral state, the periodontal disease risk, the dental caries risk, the bad breath risk, the stain risk, and the fur attachment risk, the risk increase depending on the non-practice period, and decrease by oral care.

In contrast, the gingival retraction risk increases depending on the frequency of stimulation. This means that, for the gingival retraction risk, in the care evaluation information 112 illustrated in Fig. 3, the care evaluation value in a case where oral care by teeth-brushing, which acts as a "stimulus", is practiced is indicated by a positive value, and in the non-practice evaluation information 113 illustrated in Fig. 4, the added risk is indicated by a negative value. Further, since the gingival retraction risk increases also depending on the "amount of time of single-teeth-brushing", the care evaluation value in a case where oral care by teeth-brushing is practiced may be corrected so as to increase on the basis of the amount of time of single-teeth-brushing.

### (Variation of evaluation system)

The evaluation system 100 may include a server device (not illustrated) connected to the evaluation device 1 so as to be capable of communication therewith. For example, the server device may be configured to store and manage, for each subject, information transmitted from a plurality of evaluation devices 1. In this case, for example, a healthcare professional may access subject information managed by the server device with use of a communication device, and use the information in medical practice, etc.

As an example, the server device may be implemented by cloud computing. The evaluation device 1 and the server device may be directly connected together, and may be connected so as to be capable of communication therebetween via a communication network.

### (Embodiment 2)

The evaluation device 1 in accordance with Embodiment 2 of the present invention is described here. Note that, for convenience of description, a member having the same function as a member described above is assigned the same reference sign, and the description thereof is not repeated.

In a case where record information regarding oral care cannot be obtained, an unclear period (data missing period) for which whether a subject practiced oral care is unclear is generated. For example, (1) in a case where a toothbrush different from an electric toothbrush 2 is used during a business trip, a travel, or the like, and (2) in a case where the electric toothbrush 2 is switched off and data cannot be saved, the record information is not recorded temporarily and the period during which no record information is recorded becomes the data missing period. In the data missing period, whether oral care is practiced or not cannot be identified. Thus, if a target period the oral state during which is to be evaluated includes the data missing period, it can be impossible to properly evaluate the oral state. In the evaluation device 1 in accordance with Embodiment 2, it is possible to properly evaluate the oral state of a subject even when there is the data missing period in the target period.

### (In case of presence of data missing period)

In a case where there is the data missing period in the target period, an inference section 109 infers whether the subject practiced oral care in the data missing period, and a non-practice period identifying section 103 identifies the length of a non-practice period on the basis of the result of the inference. This makes it possible to obtain a reasonable result of evaluation even if there is the data missing period.

The inference section 109 may infer whether the subject practiced oral care in the data missing period, on the basis of, for example, the oral care habits of the subject which are indicated in the record information or the oral care habits of a group of people similar to the subject.

Further, the non-practice period identifying section 103 may exclude the data missing period from the non-practice period to identify the length of the non-practice period. Specifically, after identifying the non-practice period without considering the data missing period, as in Embodiment 1, the non-practice period identifying section 103 judges whether the identified non-practice period includes the data missing period. In a case where said non-practice period includes the data missing period, the non-practice period identifying section 103 may identify the length of a period obtained by excluding the data missing period from said non-practice period, as the length of a non-practice period. This makes it possible to reduce the degree of discrepancy between the actual oral state and the result of evaluation in a case where oral care was practiced during the data missing period.

Assume, for example, that the record information indicates that, among consecutive Day 1 to Day 5, which is the target period, the subject did not practice oral care on Day 1, Day 3, and Day 5, and Day 2 and Day 4 are in the data missing period. In this case, the non-practice period identifying section 103 first identifies the length of the non-practice period as 5 days. The non-practice period identifying section 103 then excludes the data missing period of 2 days from this non-practice period to identify the length of a non-practice period as 3 days.

It is anticipated that the evaluation device 1 cannot automatically identify the data missing period. In that case, the subject may manually designate the data missing period, and the non-practice period identifying section 103 may identify the length of the non-practice period on the basis of the data missing period designated by the subject.

### [Software implementation example]

The functions of the evaluation device 1 (hereinafter, referred to as a "device") can be implemented via a program which is for causing a computer to function as the device and which is for causing a computer to function as the control blocks (in particular, the sections included in the control section 10).

In this case, the device includes, as hardware for executing the program, a computer including at least one control device (e.g., a processor) and at least one storage device (e.g., a memory). The at least one control device and the at least one storage device execute the program, so that the functions described in the above embodiments are implemented.

The program may be recorded in one or more non-transitory computer-readable recording media. The recording media may be included in the device, or need not be included in the device. In the latter case, the program may be supplied to the device via any wired or wireless transmission medium.

Furthermore, some or all of the functions of the control blocks can also be implemented by a logic circuit. For example, an integrated circuit in which logic circuits that functions as the control blocks are formed is also within the scope of the present invention. In addition, the functions of the control blocks can also be implemented by, for example, a quantum computer.

The processes described in the above embodiments may be carried out by artificial intelligence (AI). In this case, the AI may operate in the control device, or may operate in another device (e.g., an edge computer or a cloud server).

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

1: Evaluation device
2: Electric toothbrush
10: Control section
100: Evaluation system
101: Information acquisition section
102: Care evaluation section
103: Non-practice period identifying section
104: Non-practice evaluation section
105: Evaluation section
106: Prediction section
107: Measure determining section
109: Inference section
114: Result of evaluation
115: Measure information
120: Risk breakdown information

## Claims

1. An evaluation device, comprising:
a non-practice period identifying section for identifying the length of a non-practice period, which is a period during which a subject of oral state evaluation does not practice predetermined oral care; and
an evaluation section for evaluating an oral state of the subject on a basis of the length of the non-practice period.

2. The evaluation device as set forth in claim 1, further comprising:
a care evaluation section for calculating a care evaluation value indicative of an influence which practice of the predetermined oral care by the subject had on the oral state during a target period the oral state during which is to be evaluated; and
a non-practice evaluation section for calculating a non-practice evaluation value indicative of an influence which the non-practice period in the target period had on the oral state,
wherein
the evaluation section calculates an evaluation value indicative of a result of evaluation of the oral state of the subject with use of the care evaluation value and the non-practice evaluation value.

3. The evaluation device as set forth in claim 2, wherein
the care evaluation section calculates the care evaluation value depending on the predetermined oral care which the subject practiced.

4. The evaluation device as set forth in any one of claims 1 through 3, wherein
the evaluation section evaluates each of a plurality of types of risks concerning the oral state.

5. The evaluation device as set forth in any one of claims 1 through 3, wherein
the evaluation section evaluates the oral state of the subject on a basis of subject information on the subject.

6. The evaluation device as set forth in any one of claims 1 through 3, wherein
the non-practice period identifying section identifies the length of the non-practice period with respect to each of a plurality of areas into which an oral cavity of the subject is divided, and
the evaluation section evaluates each of the plurality of areas depending on the length of the non-practice period.

7. The evaluation device as set forth in any one of claims 1 through 3, further comprising
a measure determining section for determining, depending on a result of evaluation by the evaluation section, a measure to improve the result of evaluation.

8. The evaluation device as set forth in any one of claims 1 through 3, further comprising
a prediction section for predicting, on a basis of a time-series result of the oral state evaluation, a result of evaluation at a time point after a time point when said oral state evaluation was carried out.

9. The evaluation device as set forth in any one of claims 1 through 3, wherein
in a case where the non-practice period includes an unclear period during which it is unclear whether the subject practiced the predetermined oral care or not, the non-practice period identifying section excludes the unclear period from the non-practice period to identify the length of the non-practice period.

10. The evaluation device as set forth in any one of claims 1 through 3, further comprising
an inference section for inferring whether the subject practiced the predetermined oral care or not during an unclear period during which it is unclear whether the subject practiced the predetermined oral care or not,
wherein
the non-practice period identifying section identifies the length of the non-practice period on a basis of a result of the inferring.

11. An evaluation method carried out by one or more information processing devices, the evaluation method comprising:
a non-practice period identifying step of identifying the length of a non-practice period, which is a period during which a subject of oral state evaluation does not practice predetermined oral care; and
an evaluation step of calculating an evaluation value indicative of an oral state of the subject depending on the length of the non-practice period.

12. An evaluation system, comprising:
a record accumulation device for accumulating, with respect to one or more subjects of oral state evaluation, record information indicative of a record of use of a toothbrush by said one or more subjects;
an evaluation device for identifying, with respect to said one or more subjects, the length of a non-practice period, which is a period during which said one or more subjects do not practice predetermined oral care, on a basis of the record information, and evaluating an oral state depending on the length of the non-practice period; and
a presentation device for presenting feedback information depending on a result of the oral state evaluation.

13. An evaluation program for causing a computer to function as an evaluation device as set forth in claim 1, said evaluation program causing the computer to function as the non-practice period identifying section and the evaluation section.
